# EUROPÄISCHE PATENTSCHRIFT

(11) **EP 1 314 722 B1**
(45) Veröffentlichungstag und Bekanntmachung des Hinweises auf die Patenterteilung: **04.05.2005**
(21) Anmeldenummer: 01127911.4
(22) Anmeldetag: 23.11.2001
(51) Int. Cl.: C07C 309/73, C11D 1/74

(54) **Aromatische Sulfonsäurepolyglycolester**
Aromatic sulfonic acid polyglycol esters
Esters de polyglycol d'acides sulphoniques aromatiques

(43) Veröffentlichungstag der Anmeldung: 28.05.2003
(73) Patentinhaber: Cognis Iberia, S.L., 08755 Castellbisbal, (Barcelona) (ES)
(72) Erfinder: Bonastre Gilabert, Nuria, Dr., E-08210 Barbera del Vall (ES); Pi Subirana, Rafael Dr., 08400 Granollers (ES); Bigorra Llosas, Joaquin, Dr., E-08203 Sabadell (ES)
(74) Vertreter: Fabry, Bernd, Dr.

(56) Entgegenhaltungen:
- DE-A- 2 044 837

## Beschreibung

### Gebiet der Erfindung

Die Erfindung befindet sich auf dem Gebiet der nichtionischen Tenside und betrifft Anlagerungsprodukte von Alkylenoxiden an aromatische Sulfonsäuren, ein Verfahren zu deren Herstellung sowie deren Verwendung in verschiedenen Einsatzgebieten.

### Stand der Technik

Unter dem Oberbegriff "Reinigungsmittel für harte Oberflächen" werden allgemein ganz unterschiedliche Produkte verstanden. Zum einen kann es sich um typische Universalreiniger handeln, beispielsweise solche mit Hypochlorit oder Wasserstoffperoxid, die bei der Reinigung und Desinfektion im Sanitärbereich eingesetzt werden, zum anderen werden auch maschinelle Geschirrspülmittel verstanden, die entweder in Form von Pulvern oder Tablette zum Einsatz gelangen. Für die Ablösung des Schmutzes sind oberflächenaktive Substanzen verantwortlich, bei denen es sich häufig um nichtionische Tenside wie beispielsweise Anlagerungsprodukte von Alkylenoxide an Fettalkohole oder Fettsäuren, Mischether oder Hydroxymischether handelt, da diese mit den übrigen sauren oder basischen Bestandteilen der Produkte besser kompatibel sind, als beispielsweise anionische oder kationische Verbindungen. Obschon das Produktspektrum also sehr breit ist, besteht doch im Anforderungsprofil ein hohes Maß an Übereinstimmung : für die Herstellung dieser Mittel werden oberflächenaktive Mittel benötigt, die nicht nur eine ausgezeichnete Reinigungsleistung und Verträglichkeit mit anderen Rezepturbestandteilen aufweisen, sondern sich auch leicht verdicken lassen, um den Produkten eine so hohe Viskosität zu verleihen, dass sie an geneigten Flächen nicht zu rasch abfließen. Des weiteren besteht der Wunsch nach schaumarmen Produkten, vorzugsweise schaumdämpfenden Verbindungen, die die Mitverwendung von schaumstärkeren Produkten ohne Zugabe von Entschäumern erlauben. Anders ausgedrückt sollten die gesuchten Stoffe Entschäumer mit oberflächenaktiven Eigenschaften darstellen.

In diesem Zusammenhang sei auf die Deutsche Offenlegungsschrift DE-OS 2044837 (Bayer) hingewiesen, aus der Ester von aliphatischen Sulfonsäuren mit Polyethylenglykolen bekannt sind, welche als Waschrohstoffe und Emulgatoren Verwendung finden.

Die Aufgabe der vorliegenden Erfindung hat somit darin bestanden, neue nichtionische Tenside zur Verfügung zu stellen, die das eingangs geschilderte komplexe Anforderungsprofil möglichst vollständig erfüllen : hohe Reinigungsleistung, gute Kompatibilität mit sauren und basischen Rezepturbestandteilen, leichte Verdickbarkeit sowie geringes Schaumvermögen bzw. entschäumende Eigenschaften.

### Beschreibung der Erfindung

Gegenstand der Erfindung sind neue aromatische Sulfonsäurepolyglykolester der Formel **(I)**, 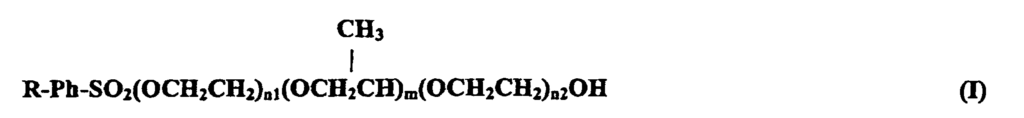 in der R für einen linearen oder verzweigten Alkylrest mit 1 bis 18 Kohlenstoffatomen, Ph für einen Phenyl- oder Naphthylrest, n1, n2 und m unabhängig voneinander für 0 der Zahlen von 1 bis 50 stehen, mit der Maßgabe, dass die Summe (n1+n2+m) von Null verschieden ist.

Überraschenderweise wurde gefunden, dass die aromatischen Sulfonsäurepolyglykolester nicht nur über ein ausgezeichnetes Reinigungsvermögen verfügen, sondern auch beispielsweise mit Hypochlorit oder Wasserstoffperoxid beliebig kompatibel sind. Die nichtionischen Tenside lassen sich leicht verdicken und sind nicht nur schaumarm, sondern besitzen im Hinblick auf andere Tenside, wie z.B. Alkylpolyglucosiden eine entschäumende Wirkung.

Ein weiterer Gegenstand der Erfindung betrifft ein Verfahren zur Herstellung von aromatischen Sulfonsäurepolyglykolestern der Formel **(I),** 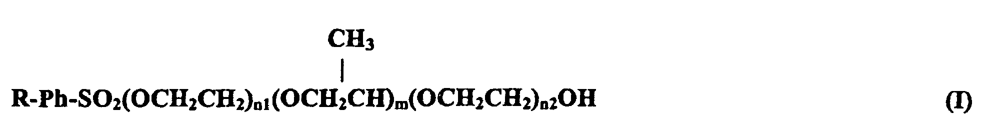 in der R für einen linearen oder verzweigten Alkylrest mit 1 bis 18 Kohlenstoffatomen, Ph für einen Phenyl- oder Naphthylrest, n1, n2 und m unabhängig voneinander für 0 der Zahlen von 1 bis 50 stehen, mit der Maßgabe, dass die Summe (n1+n2+m) von Null verschieden ist, bei dem man aromatische Sulfonsäuren der Formel **(II)**,

**R-Ph-SO**_{**3**}**H** **(II)**

in der R und Ph die gleichen Bedeutungen wie oben besitzen, mit Ethylenoxid und/oder Propylenoxid umsetzt.

### Aromatische Sulfonsäuren

Die aromatischen Sulfonsäuren, die im Sinne der Erfindung als Ausgangsstoffe in Frage kommen, weisen neben der Säuregruppe noch einen Alkylsubstituenten auf und können ihrerseits nach den einschlägigen Verfahren der präparativen organischen Chemie erhalten werden. Besonders eignet sich zu diesem Zweck die Friedel-Crafts-Alkylierung der Aromaten mit Olefinen in Gegenwart von Lewis-Säuren, wie beispielsweise Aluminium- oder Borhalogeniden, gefolgt von der Sulfonierung mit einschlägigen Sulfonierungsreagenzien, wie beispielsweise gasförmigem Schwefeltrioxid oder Chlorsulfonsäure. Die entsprechenden Produkte sind im großtechnischen Maßstab erhältlich und werden als anionische Tenside eingesetzt. Bei dem aromatischen Kern der Ausgangsstoffe kann es sich um ein Naphthalinsystem handeln, vorzugsweise liegt aber ein Benzolring vor. Demzufolge stellen die bevorzugten aromatischen Sulfonsäuren aromatische Phenylsulfonsäuren dar. Besonders bevorzugt ist der Einsatz von solchen Sulfonsäuren der Formel **(II)**, in der R für einen Alkylrest mit 10 bis 14 Kohlenstoffatomen und Ph für einen Phenylrest steht. Das wichtigste Einsatzprodukt stellt zweifellos die Dodecylbenzolsulfonsäure dar.

### Ethoxylierung

Die Ethoxylierung der aromatischen Sulfonsäuren kann in an sich bekannter Weise vorgenommen werden. Da die Stoffe selbst stark sauer sind, ist die Anwesenheit eines Katalysators zwar nicht zwingend erforderlich, eine Zugabe im Laufe der Reaktion jedoch nützlich, insbesondere dann, wenn die Säurezahl der Reaktionsmischung infolge Addition von Alkylenoxideinheiten stark abgesunken ist.. Die Anlagerung der Alkylenoxide erfolgt bei Temperaturen im Bereich von 80 bis 140 und vorzugsweise 90 bis 120 °C und bei Drücken von 1 bis 10 und vorzugsweise 1,5 bis 5 bar. Je nach Anwendungszweck hat es sich als vorteilhaft erwiesen, Produkte mit unterschiedlichen Mengen und Verteilungen - beispielsweise in Blöcken oder randomisiert - an Alkylenoxiden herzustellen, nämlich beispielsweise
- Anlagerungsprodukte von durchschnittlich 1 bis 50, vorzugsweise 5 bis 40 Mol Ethylenoxid,
- Anlagerungsprodukte von 1 bis 5, vorzugsweise 1,5 bis 2 Mol Propylenoxid und anschließend mit durchschnittlich 1 bis 50, vorzugsweise 10 bis 30 Mol Ethylenoxid umsetzt.
- Anlagerungsprodukte von Mischungen von durchschnittlich 1 bis 5 Mol Propylenoxid und 1 bis 50 Mol Ethylenoxid an die genannten Sulfonsäuren.

Insbesondere die pmpylenglykolhaltigen Ester besitzen hervorragende Eigenschaften für die Herstellung von maschinellen Geschirrspülmitteln. Im Anschluss an die Alkoxylierung empfiehlt es sich, das Reaktionsprodukt auf einen neutralen pH-Wert einzustellen, um eine spätere Hydrolyse zu verhindern.

### Gewerbliche Anwendbarkeit

Die neuen aromatischen Sulfonsäureester besitzen ausgezeichnete Reinigungs- und Netzeigenschaften, sind schaumarm bzw. entschäumend und lassen sich problemlos verdicken. Weitere Gegenstände der Erfindung betreffen ihre Verwendung sowohl in Textil- als auch Lederhilfsmitteln sowie insbesondere in Spül- und Reinigungsmitteln, speziell in Universalreinigern und maschinellen Geschirrspül- bzw. Klarspülmitteln, in denen sie in Mengen von 1 bis 50, vorzugsweise 2 bis 30 und insbesondere 10 bis 20 Gew.-% - bezogen auf die Mittel - enthalten sein können.

### Wasch-, Spül- und Reinigungsmittel

Neben den sulfonierten Sulfonsäureestern können die Wasch-, Spül- und Reinigungsmittel typische Hilfs- und Zusatzstoffe enthalten, wie beispielsweise schaumarme, vorzugsweise nichtionische Co-Tenside, Builder, öl- und fettlösende Stoffe, Bleichmittel, Bleichaktivatoren, Vergrauungsinhibitoren, Enzyme, Enzymstabilisatoren, Optische Aufheller, Polymere, Entschäumer, Sprengmittel, Duftstoffe, anorganische Salze und dergleichen, wie sie im folgenden näher erläutert werden.

### Nichtionische Co-Tenside

Besonders bevorzugt sind feste Zubereitungen, welche neben den Geminitensiden als weitere Tensidkomponente nichtionische Tenside, speziell Anlagerungsprodukte von Ethylenoxid und/oder Propylenoxid an Fett- oder Oxoalkohole enthalten. Typische Beispiele für nichtionische Co-Tenside sind Fettalkoholpolyglykolether, Alkylphenolpolyglykolether, Fettsäurepolyglykolester, Fettsäureamidpolyglykolether, Fettaminpolyglykolether, alkoxylierte Triglyceride, Mischether bzw. Mischformale, Hydroxymischether, Alk(en)yloligoglykoside, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglykoletherketten enthalten, können diese eine konventionelle, vorzugsweise jedoch eine eingeengte Homologenverteilung aufweisen. Vorzugsweise werden Fettalkoholpolyglykolether, alkoxylierte Fettsäureniedrigalkylester, Alkyloligoglucoside, Mischether und insbesondere Hydroxymischether eingesetzt.
- Die bevorzugten Fettalkoholpolyglykolether folgen der Formel **(III),**

   **R**^{**1**}**O(CH**_{**2**}**CHR**^{**2**}**O)**_{**p1**}**H (III)**

   in der R¹ für einen linearen oder verzweigten Alkyl- und/oder Alkenylrest mit 6 bis 22, vorzugsweise 12 bis 18 Kohlenstoffatomen, R² für Wasserstoff oder Methyl und p1 für Zahlen von 1 bis 20 steht. Typische Beispiele sind die Anlagerungsprodukte von durchschnittlich 1 bis 20 und vorzugsweise 5 bis 10 Mol Ethylen- und/oder Propylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Anlagerungsprodukte von 3, 5 oder 7 Mol Ethylenoxid an technische Kokosfettalkohole
- Als alkoxylierte Fettsäureniedrigalkylester kommen Tenside der Formel **(IV)** in Betracht,

   **R**^{**3**}**CO-(OCH**_{**2**}**CHR**^{**4**}**)**_{**p2**}**OR**^{**5**} **(IV)**

   in der R³CO für einen linearen oder verzweigten, gesättigten und/oder ungesättigten Acylrest mit 6 bis 22 Kohlenstoffatomen, R⁴ für Wasserstoff oder Methyl, R⁵ für lineare oder verzweigte Alkylreste mit 1 bis 4 Kohlenstoffatomen und p2 für Zahlen von 1 bis 20 steht. Typische Beispiele sind die formalen Einschubprodukte von durchschnittlich 1 bis 20 und vorzugsweise 5 bis 10 Mol Ethylen- und/oder Propylenoxid in die Methyl-, Ethyl-, Propyl-, Isopropyl-, Butyl- und tert.-Butylester von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Üblicherweise erfolgt die Herstellung der Produkte durch Insertion der Alkylenoxide in die Carbonylesterbindung in Gegenwart spezieller Katalysatoren, wie z.B. calcinierter Hydrotalcit. Besonders bevorzugt sind Umsetzungsprodukte von durchschnittlich 5 bis 10 Mol Ethylenoxid in die Esterbindung von technischen Kokosfettsäuremethylestem.
- Alkyl- und Alkenyloligoglykoside, die ebenfalls bevorzugte nichtionische Tenside darstellen, folgen üblicherweise der Formel **(V)**,

   **R**^{**6**}**O-[G]**_{**q**} **(V)**

   in der R⁶ für einen Alkyl- und/oder Alkenylrest mit 4 bis 22 Kohlenstoffatomen, G für einen Zuckerrest mit 5 oder 6 Kohlenstoffatomen und q für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP 0301298 A1** und **WO 90/03977** verwiesen. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl q in der allgemeinen Formel **(V)** gibt den Oligomerisierungsgrad (DP), d. h. die Verteilung von Mono- und Oligoglykosiden an und steht für eine Zahl zwischen 1 und 10. Während q in einer gegebenen Verbindung stets ganzzahlig sein muss und hier vor allem die Werte q = 1 bis 6 annehmen kann, ist der Wert q für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁶ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R⁶ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Weiterhin sind als nichtionische Tenside Mischether bevorzugt, die beispielsweise der Formel **(VI)** folgen,

   **R**^{**7**}**O(CH**_{**2**}**CH**_{**2**}**O)**_{**r1**}**(CH(CH**_{**3**}**)CH**_{**2**}**O)**_{**s**}**(CH**_{**2**}**CH**_{**2**}**O)**_{**r2**}**R**^{**8**} **(VI)**

   in der R⁷ für einen linearen oder verzweigten Alkyl- und/oder Alkenylrest mit 6 bis 22, vorzugsweise 12 bis 18 Kohlenstoffatomen, R⁸ für einen Alkylrest mit 1 bis 8 Kohlenstoffatomen oder einen Benzylrest, r1 und r2 unabhängig voneinander für 0 oder Zahlen von 1 bis 20 und s für 0 oder Zahlen von 0,5 bis 5 steht, mit der Maßgabe, dass die Summe (r1+r2+s) von 0 verschieden sein muss. Typische Beispiele sind etwa Kokosfettalkohol+10EO-butylether, Kokosfettalkohol+5PO+4EO-butylether oder Kokosfettalkohol+10EO-benzylether.
- Hydroxymischether (HME) stellen bekannte nichtionische Tenside mit unsymmetrischer Etherstruktur und Polyalkylenglykolanteilen dar, welche man beispielsweise erhält, indem man Olefinepoxide mit Fettalkoholpolyglykolethern einer Ringöffnungsreaktion unterwirft. Entsprechende Produkte und deren Einsatz im Bereich der Reinigung harter Oberflächen ist beispielsweise Gegenstand der europäischen Patentschrift **EP 0693049 B1** sowie der internationalen Patentanmeldung **WO 94/22800** (Olin) sowie der dort genannten Schriften. Typischerweise folgende Hydroxymischether der allgemeinen Formel **(VII),** 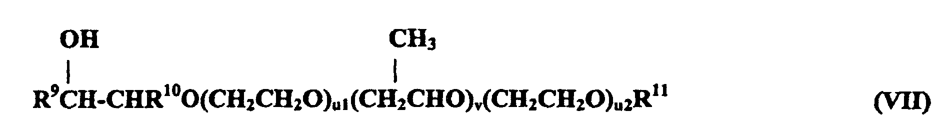 in der R⁹ für einen linearen oder verzweigten Alkylrest mit 2 bis 18, vorzugsweise 10 bis 16 Kohlenstoffatomen, R¹⁰ für Wasserstoff oder einen linearen oder verzweigten Alkylrest mit 2 bis 18 Kohlenstoffatomen, R¹¹ für einen linearen oder verzweigten Alkyl- und/oder Alkenylrest mit 1 bis 22, vorzugsweise 8 bis 18 Kohlenstoffatomen, u1 und u2 unabhängig voneinander für 0 oder Zahlen von 1 bis 60, vorzugsweise 2 bis 25 und insbesondere 5 bis 15 und v für 0 oder Zahlen von 0,5 bis 5, vorzugsweise 1 bis 2 steht, mit den Maßgaben, dass die Summe der Kohlenstoffatome in den Resten R⁹ und R¹⁰ mindestens 6 und vorzugsweise 12 bis 18 beträgt und die Summe (n1+m+n2) verschieden von 0 ist. Wie aus der Formel hervorgeht, können die HME Ringöffnungsprodukte sowohl von innenständigen Olefinen (R¹³ ungleich Wasserstoff) oder endständigen Olefinen (R¹⁰ gleich Wasserstoff) sein, wobei letztere im Hinblick auf die leichtere Herstellung und die vorteilhafteren anwendungstechnischen Eigenschaften bevorzugt sind. Gleichfalls kann der polare Teil des Moleküls eine Polyethylen- oder eine Polypropylenkette sein; ebenfalls geeignet sind gemischte Ketten von PE- und PP-Einheiten, sei es in statistischer oder Blockverteilung. Typische Beispiele sind Ringöffnungsprodukte von 1,2-Hexenepoxid, 2,3-Hexenepoxid, 1,2-Octenepoxid, 2,3-Ocetenepoxid, 3,4-Octenepoxid, 1,2-Decenepoxid, 2,3-Decenepoxid, 3,4-Decenepoxid, 4,5-Decenepoxid, 1,2-Dode-cenepoxid, 2,3-Dodecenepoxid, 3,4-Dodecenepoxid, 4,5-Dodecenepoxid, 5,6-Dodecen-epoxid, 1,2-Tetradecenepoxid, 2,3-Tetradecenepoxid, 3,4-Tetradecenepoxid, 4,5-Tetra-decenepoxid, 5,6-Tetradecenepoxid, 6,7-Tetradecenepoxid, 1,2-Hexadecen-epoxid, 2,3-Hexadecenepoxid, 3,4-Hexadecenepoxid, 4,5-Hexadecenepoxid, 5,6-Hexadecenepoxid, 6,7-Hexadecenepoxid, 7,8-Hexadecenepoxid, 1,2-Octadecenepoxid, 2,3-Octadecen-epoxid, 3,4-Octadecenepoxid, 4,5-Octadecenepoxid, 5,6-Octadecenepoxid, 6,7-Octadecenepoxid, 7,8-Octadecenepoxid und 8,9-Octadecenepoxid sowie deren Gemische mit Anlagerungsprodukten von durchschnittlich 1 bis 50, vorzugsweise 2 bis 25 und insbesondere 5 bis 15 Mol Ethylenoxid und/oder 1 bis 10, vorzugsweise 2 bis 8 und insbesondere 3 bis 5 Mol Propylenoxid an gesättigte und/oder ungesättigte primäre Alkohole mit 6 bis 22, vorzugsweise 12 bis 18 Kohlenstoffatomen, wie z.B. Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Hydroxymischether, die sich aus anwendungstechnischer Sicht als besonders geeignet erwiesen haben, folgen der Formel **(VII)** in der
   o R⁹ für einen linearen Alkylrest mit 8 bis 10 Kohlenstoffatomen, R¹⁰ für Wasserstoff, R¹¹ für einen linearen Alkylrest mit 8 bis 10 Kohlenstoffatomen, u1 für 0, v für Zahlen von 0,5 bis 2 und u2 für Zahlen von 20 bis 40 steht, wie z.B. die Handelsprodukte Dehypon® 3447 und Dehypon® 3557 der Cognis Deutschland GmbH & Co. KG;
   o R⁹ für einen linearen Alkylrest mit 8 bis 10 Kohlenstoffatomen, R¹⁰ für Wasserstoff, R¹¹ für einen verzweigten Alkylrest mit 8 bis 10 Kohlenstoffatomen, u1 und v für 0 und u2 für Zahlen von 20 bis 40 steht;
   o R⁹ für einen linearen Alkylrest mit 8 bis 10 Kohlenstoffatomen, R¹⁰ für Wasserstoff, R¹¹ für einen linearen Alkylrest mit 8 bis 10 Kohlenstoffatomen, u1 und v für 0 und u2 für Zahlen von 40 bis 60 steht.

### Builder und Co-Builder

Als Builder kommen beispielsweise Zeolithe, Alkaliphosphate, Alkalicarbonaten, Alkalisulfate, Alkalihydrogencarbonate, Alkalisilicate, Alkalicitrate, Polysaccharide und deren Derivate oder Polymere sowie deren Gemische in Frage.

Unter den Zeolithen sind die Waschmittelbuilder Zeolith A und/oder P besonders bevorzugt. Als Zeolith P wird beispielsweise Zeolith MAP^{(R)} (Handelsprodukt der Firma Crosfield) besonders bevorzugt. Geeignet sind jedoch auch Zeolith X sowie Mischungen aus A, X und/oder P wie auch Y. Von besonderem Interesse ist auch ein cokristallisiertes Natrium/Kalium-Aluminiumsilicat aus Zeolith A und Zeolith X, welches als VEGOBOND AX® (Handelsprodukt der Firma Condea Augusta S.p.A.) im Handel erhältlich ist. Der Zeolith kann als sprühgetrocknetes Pulver oder auch als ungetrocknete, von ihrer Herstellung noch feuchte, stabilisierte Suspension zum Einsatz kommen. Für den Fall, dass der Zeolith als Suspension eingesetzt wird, kann diese geringe Zusätze an nichtionischen Tensiden als Stabilisatoren enthalten, beispielsweise 1 bis 3 Gew.-%, bezogen auf Zeolith, an ethoxylierten C₁₂-C₁₈-Fettalkoholen mit 2 bis 5 Ethylenoxidgruppen, C₁₂-C₁₄-Fettalkoholen mit 4 bis 5 Ethylenoxidgruppen oder ethoxylierten Isotridecanolen. Geeignete Zeolithe weisen eine mittlere Teilchengröße von weniger als 10 µm (Volumenverteilung; Meßmethode: Coulter niger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser.

Neben den Zeolithen ist insbesondere der Einsatz der allgemein bekannten Phosphate als Trägersubstanzen möglich. Geeignet sind insbesondere die Natriumsalze der Orthophosphate, der Pyrophosphate und insbesondere der Tripolyphosphate.

Unter Alkalisilicaten kristalline, schichtförmige Alkali- und speziell Natriumsilicate der allgemeinen Formel NaMSiₓO₂ₓ₊₁·yH₂O zu verstehen, wobei M Natrium oder Wasserstoff bedeutet, x eine Zahl von 1,9 bis 4 und y eine Zahl von 0 bis 20 ist und bevorzugte Werte für x 2, 3 oder 4 sind. Derartige kristalline Schichtsilicate werden beispielsweise in der europäischen Patentanmeldung **EP 0164514 A1** beschrieben. Bevorzugte kristalline Schichtsilicate der angegebenen Formel sind solche, in denen M für Natrium steht und x die Werte 2 oder 3 annimmt. Insbesondere sind sowohl β- als auch δ-Natriumdisilicate Na₂Si₂O₅·yH₂O bevorzugt, wobei β-Natriumdisilicat beispielsweise nach dem Verfahren erhalten werden kann, das in der internationalen Patentanmeldung **WO 91/08171** beschrieben ist. Weitere geeignete Schichtsilicate sind beispielsweise aus den Patentanmeldungen **DE 2334899 A1, EP 0026529 A1** und **DE 3526405 A1** bekannt. Ihre Verwendbarkeit ist nicht auf eine spezielle Zusammensetzung bzw. Strukturformel beschränkt. Bevorzugt sind hier jedoch Smectite, insbesondere Bentonite. Geeignete Schichtsilicate, die zur Gruppe der mit Wasser quellfähigen Smectite zählen, sind z.B. solche der allgemeinen Formeln
- (OH)₄Si₈-_{y}Al_{y}(MgₓAl₄₋ₓ)O₂₀ Montmorrilonit
- (OH)₄Si_{8-y}Al_{y}(Mg_{6-z}Li_{z})O₂₀ Hectorit
- (OH)₄Si_{8-y}Al_{y}(Mg_{6-z}Al_{z})O₂₀ Saponit
mit x = 0 bis 4, y = 0 bis 2, z = 0 bis 6. Zusätzlich kann in das Kristallgitter der Schichtsilicate gemäß den vorstehenden Formeln geringe Mengen an Eisen eingebaut sein. Ferner können die Schichtsilicate aufgrund ihrer ionenaustauschenden Eigenschaften Wasserstoff-, Alkali-, Erdalkaliionen, insbesondere Na⁺ und Ca²⁺ enthalten. Die Hydratwassermenge liegt meist im Bereich von 8 bis 20 Gew.-% und ist vom Quellzustand bzw. von der Art der Bearbeitung abhängig. Brauchbare Schichtsilicate sind beispielsweise aus **US 3,966,629, US 4,062,647, EP 0026529 A1** und **EP 0028432 A1** bekannt. Vorzugsweise werden Schichtsilicate verwendet, die aufgrund einer Alkalibehandlung weitgehend frei von Calciumionen und stark färbenden Eisenionen sind. Zu den bevorzugten anorganischen Trägersubstanzen gehören auch amorphe Natriumsilicate mit einem Modul Na₂O : SiO₂ von 1 : 2 bis 1 : 3,3, vorzugsweise von 1 : 2 bis 1 : 2,8 und insbesondere von 1 : 2 bis 1 : 2,6, welche löseverzögert sind und Sekundärwascheigenschaften aufweisen. Die Löseverzögerung gegenüber herkömmlichen amorphen Natriumsilicaten kann dabei auf verschiedene Weise, beispielsweise durch Oberflächenbehandlung, Compoundierung, Kompaktierung/Verdichtung oder durch Übertrocknung hervorgerufen worden sein. Im Rahmen dieser Erfindung wird unter dem Begriff "amorph" auch "röntgenamorph" verstanden. Dies heißt, dass die Silicate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten anwendungstechnischen Eigenschaften führen, wenn die Silicatpartikel bei Elektronenbeugungsexperimenten verwaschene oder sogar scharfe Beugungsmaxima liefern. Dies ist so zu interpretieren, dass die Produkte mikrokristalline Bereiche der Größe 10 bis einige Hundert nm aufweisen, wobei Werte bis max. 50 nm und insbesondere bis max. 20 nm bevorzugt sind. Derartige sogenannte röntgenamorphe Silicate, welche ebenfalls eine Löseverzögerung gegenüber den herkömmlichen Wassergläsern aufweisen, werden beispielsweise in der deutschen Patentanmeldung **DE 4400024 A1** beschrieben. Insbesondere bevorzugt sind verdichtete/kompaktierte amorphe Silicate, compoundierte amorphe Silicate und übertrocknete röntgenamorphe Silicate.

Brauchbare organische Gerüstsubstanzen, die als Co-Builder in Frage kommen, sind beispielsweise die in Form ihrer Natriumsalze einsetzbaren Polycarbonsäuren, wie Citronensäure, Adipinsäure, Bernsteinsäure, Glutarsäure, Weinsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Auch die Säuren an sich können eingesetzt werden. Die Säuren besitzen neben ihrer Builderwirkung typischerweise auch die Eigenschaft einer Säuerungskomponente und dienen somit auch zur Einstellung eines niedrigeren und milderen pH-Wertes von Wasch- oder Reinigungsmitteln. Insbesondere sind hierbei Citronensäure, Bernsteinsäure, Glutarsäure, Adipinsäure, Gluconsäure und beliebige Mischungen aus diesen zu nennen. Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500 000. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2 000 bis 30 000. Ein bevorzugtes Dextrin ist in der britischen Patentanmeldung GB **9419091 A1** beschrieben. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Derartige oxidierte Dextrine und Verfahren ihrer Herstellung sind beispielsweise aus den europäischen Patentanmeldungen **EP 0232202 A1, EP 0427349 A1, EP 0472042 A1** und **EP 0542496 A1** sowie den internationalen Patentanmeldungen **WO 92/18542, WO 93/08251, WO 93/16110, WO 94/28030, WO 95/07303, WO 95/12619** und **WO 95/20608** bekannt. Ebenfalls geeignet ist ein oxidiertes Oligosaccharid gemäß der deutschen Patentanmeldung **DE 19600018 A1.** Ein an C₆ des Saccharidrings oxidiertes Produkt kann besonders vorteilhaft sein. Weitere geeignete Cobuilder sind Oxydisuccinate und andere Derivate von Disuccinaten, vorzugsweise Ethylendiamindisuccinat. Besonders bevorzugt sind in diesem Zusammenhang auch Glycerindisuccinate und Glycerintrisuccinate, wie sie beispielsweise in den US-amerikanischen Patentschriften US **4,524,009, US 4,639,325,** in der europäischen Patentanmeldung **EP 0150930 A1** und der japanischen Patentanmeldung **JP 93/339896** beschrieben werden. Geeignete Einsatzmengen liegen in zeolithhaltigen und/oder silicathaltigen Formulierungen bei 3 bis 15 Gew.-%.Weitere brauchbare organische Cobuilder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten. Derartige Cobuilder werden beispielsweise in der internationalen Patentanmeldung **WO 95/20029** beschrieben.

Geeignete polymere Polycarboxylate sind beispielsweise die Natriumsalze der Polyacrylsäure oder der Polymethacrylsäure, beispielsweise solche mit einer relativen Molekülmasse von 800 bis 150 000 (auf Säure bezogen und jeweils gemessen gegen Polystyrolsulfonsäure). Geeignete copolymere Polycarboxylate sind insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 5 000 bis 200 000, vorzugsweise 10 000 bis 120 000 und insbesondere 50 000 bis 100 000 (jeweils gemessen gegen Polystyrolsulfonsäure). Die (co-)polymeren Polycarboxylate können entweder als Pulver oder als wässrige Lösung eingesetzt werden, wobei 20 bis 55 Gew.-%ige wässrige Lösungen bevorzugt sind. Granulare Polymere werden zumeist nachträglich zu einem oder mehreren Basisgranulaten zugemischt. Insbesondere bevorzugt sind auch biologisch abbaubare Polymere aus mehr als zwei verschiedenen Monomereinheiten, beispielsweise solche, die gemäß der **DE 4300772 A1** als Monomere Salze der Acrylsäure und der Maleinsäure sowie Vinylalkohol bzw. Vinylalkohol-Derivate oder gemäß der **DE 4221381 C2** als Monomere Salze der Acrylsäure und der 2-Alkylallylsulfonsäure sowie Zucker-Derivate enthalten. Weitere bevorzugte Copolymere sind solche, die in den deutschen Patentanmeldungen **DE 4303320 A1** und **DE 4417734 A1** beschrieben werden und als Monomere vorzugsweise Acrolein und Acrylsäure/Acrylsäuresalze bzw. Acrolein und Vinylacetat aufweisen. Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, beispielsweise wie in der europäischen Patentanmeldung **EP 0280223 A1** beschrieben, erhalten werden können. Bevorzugte Polyacetale werden aus Dialdehyden wie Glyoxal, Glutaraldehyd, Terephthalaldehyd sowie deren Gemischen und aus Polyolcarbonsäuren

wie Gluconsäure und/oder Glucoheptonsäure erhalten.

### Öl- und fettlösende Stoffe

Zusätzlich können die Mittel auch Komponenten enthalten, welche die Öl- und Fett-Auswaschbarkeit aus Textilien positiv beeinflussen. Zu den bevorzugten öl- und fettlösenden Komponenten zählen beispielsweise nichtionische Celluloseether wie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten, insbesondere Polymere aus Ethylenterephthalaten und/oder Polyethylenglykolterephthalaten oder anionisch und/oder nichtionisch modifizierten Derivaten von diesen. Besonders bevorzugt von diesen sind die sulfonierten Derivate der Phthalsäure- und der Terephthalsäure-Polymere.

### Bleichmittel und Bleichaktivatoren

Unter den als Bleichmittel dienenden, in Wasser H₂O₂ liefernden Verbindungen haben das Natriumperborattetrahydrat und das Natriumperboratmonohydrat besondere Bedeutung. Weitere brauchbare Bleichmittel sind beispielsweise Natriumpercarbonat, Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure. Der Gehalt der Mittel an Bleichmitteln beträgt vorzugsweise 5 bis 35 Gew.-% und insbesondere bis 30 Gew.-%, wobei vorteilhafterweise Perboratmonohydrat oder Percarbonat eingesetzt wird. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und die aus den deutschen Patentanmeldungen **DE 19616693 A1** und **DE 19616767 A1** bekannten Enolester sowie acetyliertes Sorbitol und Mannitol beziehungsweise deren in der europäischen Patentanmeldung **EP 0525239 A1** beschriebene Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam, die aus den internationalen Patentanmeldungen **WO 94/27970, WO 94/28102, WO 94/28103, WO 95/00626, WO 95/14759** und **WO 95/17498** bekannt sind. Die aus der deutschen Patentanmeldung **DE 19616769 A1** bekannten hydrophil substituierten Acylacetale und die in der deutschen Patentanmeldung **DE 19616 770** sowie der internationalen Patentanmeldung **WO 95/14075** beschriebenen Acyllactame werden ebenfalls bevorzugt eingesetzt. Auch die aus der deutschen Patentanmeldung DE **4443177 A1** bekannten Kombinationen konventioneller Bleichaktivatoren können eingesetzt werden. Derartige Bleichaktivatoren sind im üblichen Mengenbereich, vorzugsweise in Mengen von 1 Gew.-% bis 10 Gew.-%, insbesondere 2 Gew.-% bis 8 Gew.-%, bezogen auf gesamtes Mittel, enthalten. Zusätzlich zu den oben aufgeführten konventionellen Bleichaktivatoren oder an deren Stelle können auch die aus den europäischen Patentschriften **EP 0446982 B1** und **EP 0453 003 B1** bekannten Sulfonimine und/oder bleichverstärkende Übergangsmetallsalze beziehungsweise Übergangsmetallkomplexe als sogenannte Bleichkatalysatoren enthalten sein. Zu den in Frage kommenden Übergangsmetallverbindungen gehören insbesondere die aus der deutschen Patentanmeldung **DE 19529905 A1** bekannten Mangan-, Eisen-, Kobalt-, Ruthenium- oder Molybdän-Selenkomplexe und deren aus der deutschen Patentanmeldung **DE 19620267 A1** bekannte N-Analogverbindungen, die aus der deutschen Patentanmeldung **DE 19536082 A1** bekannten Mangan-, Eisen-, Kobalt-, Ruthenium- oder Molybdän-Carbonylkomplexe, die in der deutschen Patentanmeldung **DE 19605688 A1** beschriebenen Mangan-, Eisen-, Kobalt-, Ruthenium-, Molybdän-, Titan-, Vanadium- und Kupfer-Komplexe mit stickstoffhaltigen Tripod-Liganden, die aus der deutschen Patentanmeldung **DE 19620411 A1** bekannten Kobalt-, Eisen-, Kupfer- und Ruthenium-Aminkomplexe, die in der deutschen Patentanmeldung **DE 4416438 A1** beschriebenen Mangan-, Kupfer- und Kobalt-Komplexe, die in der europäischen Patentanmeldung **EP 0272030 A1** beschriebenen Kobalt-Komplexe, die aus der europäischen Patentanmeldung **EP 0693550 A1** bekannten Mangan-Komplexe, die aus der europäischen Patentschrift **EP 0392592 A1** bekannten Mangan-, Eisen-, Kobalt- und Kupfer-Komplexe und/oder die in der europäischen Patentschrift **EP 0443651 B1** oder den europäischen Patentanmeldungen **EP 0458397 A1, EP 0458398 A1, EP 0549271 A1, EP 0549272 A1, EP 0544490 A1** und **EP 0544519 A1** beschriebenen Mangan-Komplexe. Kombinationen aus Bleichaktivatoren und Übergangsmetall-Bleichkatalysatoren sind beispielsweise aus der deutschen Patentanmeldung **DE 19613103 A1** und der internationalen Patentanmeldung **WO 95/27775** bekannt. Bleichverstärkende Übergangsmetallkomplexe, insbesondere mit den Zentralatomen Mn, Fe, Co, Cu, Mo, V, Ti und/oder Ru, werden in üblichen Mengen, vorzugsweise in einer Menge bis zu 1 Gew.-%, insbesondere von 0,0025 Gew.-% bis 0,25 Gew.-% und besonders bevorzugt von 0,01 Gew.-% bis 0,1 Gew.-%, jeweils bezogen auf gesamtes Mittel, eingesetzt.

### Enzyme und Enzymstabilisatoren

Als Enzyme kommen insbesondere solche aus der Klasse der Hydrolasen, wie der Proteasen, Esterasen, Lipasen bzw. lipolytisch wirkenden Enzyme, Amylasen, Cellulasen bzw. andere Glykosylhydrolasen und Gemische der genannten Enzyme in Frage. Alle diese Hydrolasen tragen in der Wäsche zur Entfernung von Verfleckungen, wie protein-, fett- oder stärkehaltigen Verfleckungen, und Vergrauungen bei. Cellulasen und andere Glykosylhydrolasen können durch das Entfernen von Pilling und Mikrofibrillen zur Farberhaltung und zur Erhöhung der Weichheit des Textils beitragen. Zur Bleiche bzw. zur Hemmung der Farbübertragung können auch Oxidoreduktasen eingesetzt werden. Besonders gut geeignet sind aus Bakterienstämmen oder Pilzen, wie Bacillus subtilis, Bacillus licheniformis, Streptomyces griseus und Humicola insolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease- und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder O-xidasen haben sich in einigen Fällen als geeignet erwiesen. Zu den geeigneten Amylasen zählen insbesondere α-Amylasen, Iso-Amylasen, Pullulanasen und Pektinasen. Als Cellulasen werden vorzugsweise Cellobiohydrolasen, Endoglucanasen und β-Glucosidasen, die auch Cellobiasen genannt werden, bzw. Mischungen aus diesen eingesetzt. Da sich die verschiedenen Cellulase-Typen durch ihre CMCase- und Avicelase-Aktivitäten unterscheiden, können durch gezielte Mischungen der Cellulasen die gewünschten Aktivitäten eingestellt werden. Die Enzyme können ihrerseits ebenfalls an Trägerstoffen adsorbiert und/oder in Hüllsubstanzen eingebettet sein, um sie gegen vorzeitige Zersetzung zu schützen. Der Anteil der Enzyme, Enzymmischungen oder Enzymgranulate kann beispielsweise etwa 0,1 bis 5 Gew.-%, vorzugsweise 0,1 bis etwa 2 Gew.-% betragen.

Zusätzlich zu den mono- und polyfunktionellen Alkoholen können die Mittel weitere Enzymstabilisatoren enthalten. Beispielsweise können 0,5 bis 1 Gew.-% Natriumformiat eingesetzt werden. Möglich ist auch der Einsatz von Proteasen, die mit löslichen Calciumsalzen und einem Calciumgehalt von vorzugsweise etwa 1,2 Gew.-%, bezogen auf das Enzym, stabilisiert sind. Außer Calciumsalzen dienen auch Magnesiumsalze als Stabilisatoren. Besonders vorteilhaft ist jedoch der Einsatz von Borverbindungen, beispielsweise von Borsäure, Boroxid, Borax und anderen Alkalimetallboraten wie den Salzen der Orthoborsäure (H₃BO₃), der Metaborsäure (HBO₂) und der Pyroborsäure (Tetraborsäure H₂B₄O₇).

### Vergrauungsinhibitoren

Vergrauungsinhibitoren haben die Aufgabe, den von der Faser abgelösten Schmutz in der Flotte suspendiert zu halten und so das Wiederaufziehen des Schmutzes zu verhindern. Hierzu sind wasserlösliche Kolloide meist organischer Natur geeignet, beispielsweise die wasserlöslichen Salze polymerer Carbonsäuren, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, z.B. abgebaute Stärke, Aldehydstärken usw.. Auch Polyvinylpyrrolidon ist brauchbar. Bevorzugt werden jedoch Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, sowie Polyvinylpyrrolidon beispielsweise in Mengen von 0,1 bis 5 Gew.-%, bezogen auf die Mittel, eingesetzt.

### Optische Aufheller

Die Mittel können als optische Aufheller Derivate der Diaminostilbendisulfonsäure bzw. deren Alkalimetallsalze enthalten. Geeignet sind z.B. Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, z.B. die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten Aufheller können verwendet werden. Einheitlich weiße Granulate werden erhalten, wenn die Mittel außer den üblichen Aufhellern in üblichen Mengen, beispielsweise zwischen 0,1 und 0,5 Gew.-%, vorzugsweise zwischen 0,1 und 0,3 Gew.-%, auch geringe Mengen, beispielsweise 10⁻⁶ bis 10⁻³ Gew.-%, vorzugsweise um 10⁻⁵ Gew.-%, eines blauen Farbstoffs enthalten. Ein besonders bevorzugter Farbstoff ist Tinolux® (Handelsprodukt der Ciba-Geigy).

### Polymere

Als schmutzabweisende Polymere ("soil repellants") kommen solche Stoffe in Frage, die vorzugsweise Ethylenterephthalat- und/oder Polyethylenglykolterephthalatgruppen enthalten, wobei das Molverhältnis Ethylenterephthalat zu Polyethylenglykolterephthalat im Bereich von 50 : 50 bis 90 : 10 liegen kann. Das Molekulargewicht der verknüpfenden Polyethylenglykoleinheiten liegt insbesondere im Bereich von 750 bis 5000, d.h., der Ethoxylierungsgrad der Polyethylenglykolgruppenhaltigen Polymere kann ca. 15 bis 100 betragen. Die Polymeren zeichnen sich durch ein durchschnittliches Molekulargewicht von etwa 5000 bis 200.000 aus und können eine Block-, vorzugsweise aber eine Random-Struktur aufweisen. Bevorzugte Polymere sind solche mit Molverhältnissen Ethylenterephthalat/Polyethylenglykolterephthalat von etwa 65 : 35 bis etwa 90 : 10, vorzugsweise von etwa 70 : 30 bis 80 : 20. Weiterhin bevorzugt sind solche Polymeren, die verknüpfende Polyethylenglykoleinheiten mit einem Molekulargewicht von 750 bis 5000, vorzugsweise von 1000 bis etwa 3000 und ein Molekulargewicht des Polymeren von etwa 10.000 bis etwa 50.000 aufweisen. Beispiele für handelsübliche Polymere sind die Produkte Milease® T (ICI) oder Repelotex® SRP 3 (Rhône-Poulenc).

### Entschäumer

Als Entschäumer können wachsartige Verbindungen eingesetzt werden. Als "wachsartig" werden solche Verbindungen verstanden, die einen Schmelzpunkt bei Atmosphärendruck über 25 °C (Raumtemperatur), vorzugsweise über 50 °C und insbesondere über 70 °C aufweisen. Die wachsartigen Entschäumersubstanzen sind in Wasser praktisch nicht löslich, d.h. bei 20 °C weisen sie in 100 g Wasser eine Löslichkeit unter 0,1 Gew.-% auf. Prinzipiell können alle aus dem Stand der Technik bekannten wachsartigen Entschäumersubstanzen enthalten sein. Geeignete wachsartige Verbindungen sind beispielsweise Bisamide, Fettalkohole, Fettsäuren, Carbonsäureester von ein- und mehrwertigen Alkoholen sowie Paraffinwachse oder Mischungen derselben. Alternativ können natürlich auch die für diesen Zweck bekannten Silikonverbindungen eingesetzt werden.

Geeignete Paraffinwachse stellen im allgemeinen ein komplexes Stoffgemisch ohne scharfen Schmelzpunkt dar. Zur Charakterisierung bestimmt man üblicherweise seinen Schmelzbereich durch Differential-Thermo-Analyse (DTA), wie in **"The Analyst"** **87** **(1962), 420,** beschrieben, und/oder seinen Erstarrungspunkt. Darunter versteht man die Temperatur, bei der das Paraffin durch langsames Abkühlen aus dem flüssigen in den festen Zustand übergeht. Dabei sind bei Raumtemperatur vollständig flüssige Paraffine, das heißt solche mit einem Erstarrungspunkt unter 25 °C, erfindungsgemäß nicht brauchbar. Zu den Weichwachsen, die einen Schmelzpunkt im Bereich von 35 bis 50 °C aufweisen, zählen vorzugsweise der Gruppe der Petrolate und deren Hydrierprodukte. Sie setzen sich aus mikrokristallinen Paraffinen und bis zu 70 Gew.-% Öl zusammen, besitzen eine salbenartige bis plastisch feste Konsistenz und stellen bitumenfreie Rückstände aus der Erdölverarbeitung dar. Besonders bevorzugt sind Destillationsrückstände (Petrolatumstock) bestimmter paraffinbasischer und gemischtbasischer Rohöle, die zu Vaseline weiterverarbeitet werden. Vorzugsweise handelt es sich weiterhin um aus Destillationsrückständen paraffin- und gemischtbasyischer Rohöle und Zylinderöldestillate mittels Lösungsmittel abgeschiedene bitumenfreie, ölartige bis feste Kohlenwasserstoffe. Sie sind von halbfester, zügiger, klebriger bis plastisch-fester Konsistenz und besitzen Schmelzpunkte zwischen 50 und 70 °C. Diese Petrolate stellen die wichtigste Ausgangsbasis für die Herstellung von Mikrowachsen dar. Weiterhin geeignet sind die aus hochviskosen, paraffinhaltigen Schmieröldestillaten bei der Entparaffinierung abgeschiedenen festen Kohlenwasserstoffen mit Schmelzpunkten zwischen 63 und 79 °C. Bei diesen Petrolaten handelt es sich um Gemische aus mikrokristallinen Wachsen und hochschmelzenden n-Paraffinen. Eingesetzt werden können beispielsweise die aus **EP 0309931 A1** bekannten Paraffinwachsgemische aus beispielsweise 26 Gew.-% bis 49 Gew.-% mikrokristallinem Paraffinwachs mit einem Erstarrungspunkt von 62 °C bis 90 °C, 20 Gew.-% bis 49 Gew.-% Hartparaffin mit einem Erstarrungspunkt von 42 °C bis 56 °C und 2 Gew.-% bis 25 Gew.-% Weichparaffin mit einem Erstarrungspunkt von 35 °C bis 40 °C. Vorzugsweise werden Paraffine bzw. Paraffingemische verwendet, die im Bereich von 30 °C bis 90 °C erstarren. Dabei ist zu beachten, dass auch bei Raumtemperatur fest erscheinende Paraffinwachsgemische unterschiedliche Anteile an flüssigem Paraffin enthalten können. Bei den erfindungsgemäß brauchbaren Paraffinwachsen liegt dieser Flüssiganteil so niedrig wie möglich und fehlt vorzugsweise ganz. So weisen besonders bevorzugte Paraffinwachsgemische bei 30 °C einen Flüssiganteil von unter 10 Gew.-%, insbesondere von 2 Gew.-% bis 5 Gew.-%, bei 40 °C einen Flüssiganteil von unter 30 Gew.-%, vorzugsweise von 5 Gew.-% bis 25 Gew.-% und insbesondere von 5 Gew.-% bis 15 Gew.-%, bei 60 °C einen Flüssiganteil von 30 Gew.-% bis 60 Gew.-%, insbesondere von 40 Gew.-% bis 55 Gew.-%, bei 80 °C einen Flüssiganteil von 80 Gew.-% bis 100 Gew.-%, und bei 90 °C einen Flüssiganteil von 100 Gew.-% auf. Die Temperatur, bei der ein Flüssiganteil von 100 Gew.-% des Paraffinwachses erreicht wird, liegt bei besonders bevorzugten Paraffinwachsgemischen noch unter 85 °C, insbesondere bei 75 °C bis 82 °C. Bei den Paraffinwachsen kann es sich um Petrolatum, mikrokristalline Wachse bzw. hydrierte oder partiell hydrierte Paraffinwachse handeln.

Geeignete Bisamide als Entschäumer sind solche, die sich von gesättigten Fettsäuren mit 12 bis 22, vorzugsweise 14 bis 18 C-Atomen sowie von Alkylendiaminen mit 2 bis 7 C-Atomen ableiten. Geeignete Fettsäuren sind Laurin-, Myristin-, Stearin-, Arachin- und Behensäure sowie deren Gemische, wie sie aus natürlichen Fetten beziehungsweise gehärteten Ölen, wie Talg oder hydriertem Palmöl, erhältlich sind. Geeignete Diamine sind beispielsweise Ethylendiamin, 1,3-Propylendiamin, Tetramethylendiamin, Pentamethylendiamin, Hexamethylendiamin, p-Phenylendiamin und Toluylendiamin. Bevorzugte Diamine sind Ethylendiamin und Hexamethylendiamin. Besonders bevorzugte Bisamide sind Bismyristoylethylendiamin, Bispalmitoylethylendiamin, Bisstearoylethylendiamin und deren Gemische sowie die entsprechenden Derivate des Hexamethylendiamins.

Geeignete Carbonsäureester als Entschäumer leiten sich von Carbonsäuren mit 12 bis 28 Kohlenstoffatomen ab. Insbesondere handelt es sich um Ester von Behensäure, Stearinsäure, Hydroxystearinsäure, Ölsäure, Palmitinsäure, Myristinsäure und/oder Laurinsäure. Der Alkoholteil des Carbonsäureesters enthält einen ein- oder mehrwertigen Alkohol mit 1 bis 28 Kohlenstoffatomen in der Kohlenwasserstoffkette. Beispiele von geeigneten Alkoholen sind Behenylalkohol, Arachidylalkohol, Kokosalkohol, 12-Hydroxystearylalkohol, Oleylalkohol und Laurylalkohol sowie Ethylenglykol, Glycerin, Polyvinylalkohol, Saccharose, Erythrit, Pentaerythrit, Sorbitan und/oder Sorbit. Bevorzugte Ester sind solche von Ethylenglykol, Glycerin und Sorbitan, wobei der Säureteil des Esters insbesondere aus Behensäure, Stearinsäure, Ölsäure, Palmitinsäure oder Myristinsäure ausgewählt wird. In Frage kommende Ester mehrwertiger Alkohole sind beispielsweise Xylitmonopalmitat, Pentarythritmonostearat, Glycerinmonostearat, Ethylenglykolmonostearat und Sorbitanmonostearat, Sorbitanpalmitat, Sorbitanmonolaurat, Sorbitandilaurat, Sorbitandistearat, Sorbitandibehenat, Sorbitandioleat sowie gemischte Talgalkylsorbitanmono- und -diester. Brauchbare Glycerinester sind die Mono-, Di- oder Triester von Glycerin und genannten Carbonsäuren, wobei die Mono- oder Dieester bevorzugt sind. Glycerinmonostearat, Glycerinmonooleat, Glycerinmonopalmitat, Glycerinmonobehenat und Glycerindistearat sind Beispiele hierfür. Beispiele für geeignete natürliche Ester als Entschäumer sind Bienenwachs, das hauptsächlich aus den Estern CH₃(CH₂)₂₄COO(CH₂)₂₇CH₃ und CH₃(CH₂)₂₆COO(CH₂)₂₅CH₃ besteht, und Carnaubawachs, das ein Gemisch von Carnaubasäurealkylestern, oft in Kombination mit geringen Anteilen freier Carnaubasäure, weiteren langkettigen Säuren, hochmolekularen Alkoholen und Kohlenwasserstoffen, ist.

Geeignete Carbonsäuren als weitere Entschäumerverbindung sind insbesondere Behensäure, Stearinsäure, Ölsäure, Palmitinsäure, Myristinsäure und Laurinsäure sowie deren Gemische, wie sie aus natürlichen Fetten bzw. gegebenenfalls gehärteten Ölen, wie Talg oder hydriertem Palmöl, erhältlich sind. Bevorzugt sind gesättigte Fettsäuren mit 12 bis 22, insbesondere 18 bis 22 C-Atomen. In gleicher Weise können die entsprechenden Fettalkohole gleicher C-Kettenlänge eingesetzt werden.

Weiterhin können zusätzlich Dialkylether als Entschäumer enthalten sein. Die Ether können asymmetrisch oder aber symmetrisch aufgebaut sein, d.h. zwei gleiche oder verschiedene Alkylketten, vorzugsweise mit 8 bis 18 Kohlenstoffatomen enthalten. Typische Beispiele sind Di-n-octylether, Di-i-octylether und Di-n-stearylether, insbesondere geeignet sind Dialkylether, die einen Schmelzpunkt über 25 °C, insbesondere über 40 °C aufweisen.

Weitere geeignete Entschäumerverbindungen sind Fettketone, die nach den einschlägigen Methoden der präparativen organischen Chemie erhalten werden können. Zu ihrer Herstellung geht man beispielsweise von Carbonsäuremagnesiumsalzen aus, die bei Temperaturen oberhalb von 300 °C unter Abspaltung von Kohlendioxid und Wasser pyrolysiert werden, beispielsweise gemäß der deutschen Offenlegungsschrift **DE 2553900 OS**. Geeignete Fettketone sind solche, die durch Pyrolyse der Magnesiumsalze von Laurinsäure, Myristinsäure, Palmitinsäure, Palmitoleinsäure, Stearinsäure, Ölsäure, Elaidinsäure, Petroselinsäure, Arachinsäure, Gadoleinsäure, Behensäure oder Erucasäure hergestellt werden.

Weitere geeignete Entschäumer sind Fettsäurepolyethylenglykolester, die vorzugsweise durch basisch homogen katalysierte Anlagerung von Ethylenoxid an Fettsäuren erhalten werden. Insbesondere erfolgt die Anlagerung von Ethylenoxid an die Fettsäuren in Gegenwart von Alkanolaminen als Katalysatoren. Der Einsatz von Alkanolaminen, speziell Triethanolamin, führt zu einer äußerst selektiven Ethoxylierung der Fettsäuren, insbesondere dann, wenn es darum geht, niedrig ethoxylierte Verbindungen herzustellen. Innerhalb der Gruppe der Fettsäurepolyethylenglykolester werden solche bevorzugt, die einen Schmelzpunkt über 25 °C, insbesondere über 40 °C aufweisen .

Innerhalb der Gruppe der wachsartigen Entschäumer werden besonders bevorzugt die beschriebenen Paraffinwachse alleine als wachsartige Entschäumer eingesetzt oder in Mischung mit einem der anderen wachsartigen Entschäumer, wobei der Anteil der Paraffinwachse in der Mischung vorzugsweise über 50 Gew.-% - bezogen auf wachsartige Entschäumermischung - ausmacht. Die Paraffinwachse können bei Bedarf auf Träger aufgebracht sein. Als Trägermaterial sind alle bekannten anorganischen und/oder organischen Trägermaterialien geeignet. Beispiele für typische anorganische Trägermaterialien sind Alkalicarbonate, Alumosilicate, wasserlösliche Schichtsilicate, Alkalisilicate, Alkalisulfate, beispielsweise Natriumsulfat, und Alkaliphosphate. Bei den Alkalisilicaten handelt es sich vorzugsweise um eine Verbindung mit einem Molverhältnis Alkalioxid zu SiO₂ von 1 : 1,5 bis 1 : 3,5. Die Verwendung derartiger Silicate resultiert in besonders guten Korneigenschaften, insbesondere hoher Abriebsstabilität und dennoch hoher Auflösungsgeschwindigkeit in Wasser. Zu den als Trägermaterial bezeichneten Alumosilicaten gehören insbesondere die Zeolithe, beispielsweise Zeolith NaA und NaX. Zu den als wasserlöslichen Schichtsilicaten bezeichneten Verbindungen gehören beispielsweise amorphes oder kristallines Wasserglas. Weiterhin können Silicate Verwendung finden, welche unter der Bezeichnung Aerosil® oder Sipernat® im Handel sind. Als organische Trägermaterialien kommen zum Beispiel filmbildende Polymere, beispielsweise Polyvinylalkohole, Polyvinylpyrrolidone, Poly(meth)acrylate, Polycarboxylate, Cellulosederivate und Stärke in Frage. Brauchbare Celluloseether sind insbesondere Alkalicarboxymethylcellulose, Methylcellulose, Ethylcellulose, Hydroxyethylcellulose und sogenannte Cellulosemischether, wie zum Beispiel Methylhydroxyethylcellulose und Methylhydroxypropylcellulose, sowie deren Mischungen. Besonders geeignete Mischungen sind aus Natrium-Carboxymethylcellulose und Methylcellulose zusammengesetzt, wobei die Carboxymethylcellulose üblicherweise einen Substitutionsgrad von 0,5 bis 0,8 Carboxymethylgruppen pro Anhydroglukoseeinheit und die Methylcellulose einen Substitutionsgrad von 1,2 bis 2 Methylgruppen pro Anhydroglukoseeinheit aufweist. Die Gemische enthalten vorzugsweise Alkalicarboxymethylcellulose und nichtionischen Celluloseether in Gewichtsverhältnissen von 80 : 20 bis 40 : 60, insbesondere von 75 : 25 bis 50 : 50. Als Träger ist auch native Stärke geeignet, die aus Amylose und Amylopectin aufgebaut ist. Als native Stärke wird Stärke bezeichnet, wie sie als Extrakt aus natürlichen Quellen zugänglich ist, beispielsweise aus Reis, Kartoffeln, Mais und Weizen. Native Stärke ist ein handelsübliches Produkt und damit leicht zugänglich. Als Trägermaterialien können einzeln oder mehrere der vorstehend genannten Verbindungen eingesetzt werden, insbesondere ausgewählt aus der Gruppe der Alkalicarbonate, Alkalisulfate, Alkaliphosphate, Zeolithe, wasserlösliche Schichtsilicate, Alkalisilicate, Polycarboxylate, Celluloseether, Polyacrylat/Polymethacrylat und Stärke. Besonders geeignet sind Mischungen von Alkalicarbonaten, insbesondere Natriumcarbonat, Alkalisilicaten, insbesondere Natriumsilicat, Alkalisulfaten, insbesondere Natriumsulfat und Zeolithen.

Geeignete Silicone sind übliche Organopolysiloxane, die einen Gehalt an feinteiliger Kieselsäure, die wiederum auch silaniert sein kann, aufweisen können. Derartige Organopolysiloxane sind beispielsweise in der Europäischen Patentanmeldung **EP 0496510 A1** beschrieben. Besonders bevorzugt sind Polydiorganosiloxane und insbesondere Polydimethylsiloxane, die aus dem Stand der Technik bekannt sind. Geeignete Polydiorganosiloxane weisen eine nahezu lineare Kette auf und weisen einen Oligomerisierungsgrad von 40 bis 1500 auf. Beispiele für geeignete Substituenten sind Methyl, Ethyl, Propyl, Isobutyl, tert. Butyl und Phenyl. Weiterhin geeignet sind amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. In der Regel enthalten die Silicone im allgemeinen und die Polydiorganosiloxane im besonderen feinteilige Kieselsäure, die auch silaniert sein kann. Insbesondere geeignet sind im Sinne der vorliegenden Erfindung kieselsäurehaltige Dimethylpolysiloxane. Vorteilhafterweise haben die Polydiorganosiloxane eine Viskosität nach Brookfield bei 25 °C (Spindel 1, 10 Upm) im Bereich von 5000 mPas bis 30 000 mPas, insbesondere von 15 000 bis 25 000 mPas. Vorzugsweise werden die Silicone in Form ihrer wässrigen Emulsionen eingesetzt. In der Regel gibt man das Silicon zu vorgelegtem Wasser unter Rühren. Falls gewünscht kann man zur Erhöhung der Viskosität der wässrigen Siliconemulsionen Verdickungsmittel, wie sie aus dem Stand der Technik bekannt sind, zugeben. Diese können anorganischer und/oder organischer Natur sein, besonders bevorzugt werden nichtionische Celluloseether wie Methylcellulose, Ethylcellulose und Mischether wie Methylhydoxyethylcellulose, Methylhydroxypropylcellulose, Methylhydroxybutylcellulose sowie anionische Carboxycellulose-Typen wie das Carboxymethylcellulose-Natriumsalz (Abkürzung CMC). Insbesondere geeignete Verdicker sind Mischungen von CMC zu nichtionischen Celluloseethern im Gewichtsverhältnis 80 : 20 bis 40 : 60, insbesondere 75 : 25 bis 60 : 40. In der Regel und besonders bei Zugabe der beschriebenen Verdickermischungen empfehlen sich Einsatzkonzentrationen von cirka 0,5 bis 10, insbesondere von 2,0 bis 6 Gew.-% - berechnet als Verdickermischung und bezogen auf wässrige Siliconemulsion. Der Gehalt an Siliconen der beschriebenen Art in den wässrigen Emulsionen liegt vorteilhafterweise im Bereich von 5 bis 50 Gew.%, insbesondere von 20 bis 40 Gew.-% - berechnet als Silicone und bezogen auf wässrige Siliconemulsion. Nach einer weiteren vorteilhaften Ausgestaltung erhalten die wässrigen Siliconlösungen als Verdicker Stärke, die aus natürlichen Quellen zugänglich ist, beispielsweise aus Reis, Kartoffeln, Mais und Weizen. Die Stärke ist vorteilhafterweise in Mengen von 0,1 bis zu 50 Gew.-% - bezogen auf Silicon-Emulsion - enthalten und insbesondere in Mischung mit den schon beschriebenen Verdickermischungen aus NatriumCarboxymethylcellulose und einem nichtionischen Celluloseether in den schon genannten Mengen. Zur Herstellung der wässrigen Siliconemulsionen geht man zweckmäßigerweise so vor, dass man die gegebenenfalls vorhandenen Verdickungsmittel in Wasser vorquellen lässt, bevor die Zugabe der Silicone erfolgt. Das Einarbeiten der Silicone erfolgt zweckmäßigerweise mit Hilfe wirksamer Rühr- und Mischungsvorrichtungen.

### Sprengmittel

Die festen Zubereitungen können des weiteren Spreng- oder Desintegrationsmittel enthalten. Hierunter sind Stoffe zu verstehen, die den Formkörpern zugegeben werden, um deren Zerfall beim Inkontaktbringen mit Wasser zu beschleunigen. Übersichten hierzu finden sich z.B. in **J.Pharm.Sci.** **61** **(1972), Römpp Chemilexikon, 9. Auflage, Band 6, S. 4440** sowie und **Voigt** ***"Lehrbuch der pharmazeutischen Technologie***" **(6. Auflage, 1987, S. 182-184).** Diese Stoffe vergrößern bei Wasserzutritt ihr Volumen, wobei einerseits das Eigenvolumen vergrößert (Quellung), andererseits auch über die Freisetzung von Gasen ein Druck erzeugt werden kann, der die Tablette in kleinere Partikel zerfallen lässt. Altbekannte Desintegrationshilfsmittel sind beispielsweise Carbonat/Citronensäure-Systeme, wobei auch andere organische Säuren eingesetzt werden können. Quellende Desintegrationshilfsmittel sind beispielsweise synthetische Polymere wie gegebenenfalls quervernetztes Polyvinylpyrrolidon (PVP) oder natürliche Polymere bzw. modifizierte Naturstoffe wie Cellulose und Stärke und ihre Derivate, Alginate oder Casein-Derivate. Als bevorzugte Desintegrationsmittel werden im Rahmen der vorliegenden Erfindung Desintegrationsmittel auf Cellulosebasis eingesetzt. Reine Cellulose weist die formale Bruttozusammensetzung (C₆H₁₀O₅)ₙ auf und stellt formal betrachtet ein β-1,4-Polyacetal von Cellobiose dar, die ihrerseits aus zwei Molekülen Glucose aufgebaut ist. Geeignete Cellulosen bestehen dabei aus ca. 500 bis 5000 Glucose-Einheiten und haben demzufolge durchschnittliche Molmassen von 50.000 bis 500.000. Als Desintegrationsmittel auf Cellulosebasis verwendbar sind im Rahmen der vorliegenden Erfindung auch Cellulose-Derivate, die durch polymeranaloge Reaktionen aus Cellulose erhältlich sind. Solche chemisch modifizierten Cellulosen umfassen dabei beispielsweise Produkte aus Veresterungen bzw. Veretherungen, in denen Hydroxy-Wasserstoffatome substituiert wurden. Aber auch Cellulosen, in denen die Hydroxylgruppen gegen funktionelle Gruppen, die nicht über ein Sauerstoffatom gebunden sind, ersetzt wurden, lassen sich als Cellulosederivate einsetzen. In die Gruppe der Cellulosederivate fallen beispielsweise Alkalicellulosen, Carboxymethylcellulose (CMC), Celluloseester und -ether sowie Aminocellulosen. Die genannten Cellulosederivate werden vorzugsweise nicht allein als Sprengmittel auf Cellulosebasis eingesetzt, sondern in Mischung mit Cellulose verwendet. Der Gehalt dieser Mischungen an Cellulosederivaten beträgt vorzugsweise unterhalb 50 Gew.-%, besonders bevorzugt unterhalb 20 Gew.-%, bezogen auf das Desintegrationsmittel auf Cellulosebasis. Besonders bevorzugt wird als Desintegrationsmittel auf Cellulosebasis reine Cellulose eingesetzt, die frei von Cellulosederivaten ist. Als weiteres Desintegrationsmittel auf Cellulosebasis oder als Bestandteil dieser Komponente kann mikrokristalline Cellulose verwendet werden. Diese mikrokristalline Cellulose wird durch partielle Hydrolyse von Cellulosen unter solchen Bedingungen erhalten, die nur die amorphen Bereiche (ca. 30% der Gesamt-Cellulosemasse) der Cellulosen angreifen und vollständig auflösen, die kristallinen Bereiche (ca. 70%) aber unbeschadet lassen. Eine nachfolgende Desaggregation der durch die Hydrolyse entstehenden mikrofeinen Cellulosen liefert die mikrokristallinen Cellulosen, die Primärteilchengrößen von ca. 5 µm aufweisen und beispielsweise zu Granulaten mit einer mittleren Teilchengröße von 200 µm kompaktierbar sind. Die Sprengmittel können im Formkörper makroskopisch betrachtet homogen verteilt vorliegen, mikroskopisch gesehen bilden sie jedoch herstellungsbedingt Zonen erhöhter Konzentration. Sprengmittel, die im Sinne der Erfindung zugegen sein können, wie z.B. Kollidon, Alginsäure und deren Alkalisalze, amorphe oder auch teilweise kristalline Schichtsilicate (Bentonite), Polyacrylate, Polyethylenglykole sind beispielsweise den Druckschriften **WO 98/40462** (Rettenmaier), **WO 98/55583** und **WO 98/55590** (Unilever) und **WO 98/40463, DE 19709991** und **DE 19710254** (Henkel) zu entnehmen. Auf die Lehre dieser Schriften wird ausdrücklich Bezug genommen. Die Formkörper können die Sprengmittel in Mengen von 0,1 bis 25, vorzugsweise 1 bis 20 und insbesondere 5 bis 15 Gew.-% - bezogen auf die Formkörper enthalten.

### Duftstoffe

Als Parfümöle bzw. Duftstoffe können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Die Duftstoffe können direkt in die erfindungsgemäßen Mittel eingearbeitet werden, es kann aber auch vorteilhaft sein, die Duftstoffe auf Träger aufzubringen, welche die Haftung des Parfüms auf der Wäsche verstärken und durch eine langsamere Duftfreisetzung für langanhaltenden Duft der Textilien sorgen. Als solche Trägermaterialien haben sich beispielsweise Cyclodextrine bewährt, wobei die Cyclodextrin-Parfüm-Komplexe zusätzlich noch mit weiteren Hilfsstoffen beschichtet werden können.

### Anorganische Salze

Weitere geeignete Inhaltsstoffe der Mittel sind wasserlösliche anorganische Salze wie Bicarbonate, Carbonate, amorphe Silicate, normale Wassergläser, welche keine herausragenden Buildereigenschaften aufweisen, oder Mischungen aus diesen; insbesondere werden Alkalicarbonat und/oder amorphes Alkalisilicat, vor allem Natriumsilicat mit einem molaren Verhältnis Na₂O : SiO₂ von 1 : 1 bis 1 : 4,5, vorzugsweise von 1 : 2 bis 1 : 3,5, eingesetzt. Der Gehalt an Natriumcarbonat in den Endzubereitungen beträgt dabei vorzugsweise bis zu 40 Gew.-%, vorteilhafterweise zwischen 2 und 35 Gew.-%. Der Gehalt der Mittel an Natriumsilicat (ohne besondere Buildereigenschaften) beträgt im allgemeinen bis zu 10 Gew.-% und vorzugsweise zwischen 1 und 8 Gew.-%. Als Füll- bzw. Stellmittel kann ferner beispielsweise Natriumsulfat in Mengen von 0 bis 10, insbesondere 1 bis 5 Gew.-% - bezogen auf Mittel - enthalten sein.

### Beispiele

### Beispiel 1

In einem Rührautoklaven wurden 877 g (2,89 Mol) Dodecylbenzolsulfonsäure vorgelegt. Anschließend wurde der Reaktor abwechselnd dreimal evakuiert und mit Stickstoffgespült und schließlich bei einer Temperatur von 97 °C mit 6 Mol Ethylenoxid beaufschlagt, wobei der Druck bis auf 1 bar anstieg. Der Verlauf der Reaktion wurde über Probennahme verfolgt. Nachdem die Säurezahl auf einen Wert unter 1 mg KOH/g abgefallen war, wurde der Autoklav abgekühlt, entspannt und der pH-Wert des Reaktionsproduktes durch Zugabe von Diethanolamin neutral eingestellt. Die resultierende ethoxylierte Dodecylbenzolsulfonsäure wies einen durchschnittlichen Ethoxylierungsgrad von 2,1 auf und lag als transparente, gelblich gefärbte Flüssigkeit vor.

### Beispiel 2

Analog Beispiel 1 wurden 619 g (2 Mol) Dodecylbenzolsulfonsäure vorgelegt und mit Ethylenoxid zur Reaktion gebracht. Nach Erreichen einer Säurezahl kleiner 1 wurden 2 g einer 50 Gew.-%igen Natriumhydroxidlösung zugegeben und Vakuum angelegt, um Wasserspuren zu entfernen. Anschließend wurde die Temperatur auf 120 bis 135 °C gesteigert und weiteres Ethylenoxid aufgegeben, wobei der Druck bis auf 4,5 bar anstieg. Insgesamt betrug der Verbrauch an Ethylenoxid 675 g (15,3 Mol). Die resultierende ethoxylierte Dodecylbenzolsulfonsäure wies einen durchschnittlichen Ethoxylierungsgrad von 7,5 auf und lag als transparente, gelblich gefärbte Flüssigkeit vor.

### Anwendungstechnische Beispiele

Die Leistung der erfindungsgemäßen aromatischen Sulfonsäureester wurde gegenüber nichtionischen Tensiden des Handels vom Typ der PO/EO-Anlagerungsprodukte an Fettalkohole untersucht. Dabei wurden die Tenside sowohl alleine als auch in einer alkalischen Reinigerformulierung getestet. Zur Bestimmung der Reinigungsleistung wurde eine Keramikoberfläche mit einer Mischung aus Fett und Russ angeschmutzt, mit 10 ml der Testmischungen gereinigt und einem feuchten Lappen nachgewischt. Die Beurteilung der Reinigungsleistung erfolgte subjektiv auf einer Skala von (++) = schlierenfrei sauber; (+) = sauber, aber Schlieren; (o) = geringe Rückstände; (-) deutliche Rückstände. Die Messung der Viskosität erfolgte nach der Brookfield-Methode (20 °C, Spindel 1, 10 Upm), die Bestimmung des Schaumvermögens nach dem Ross-Miles Test bei 20 °C und einer Wasserhärte von 16 °dH. Die Ergebnisse sind in Tabelle 1 zusammengefasst. Die Beispiele 1 und 2 sind erfindungsgemäß, die Beispiele V1 bis V3 dienen zum Vergleich.

**Tabelle 1**

| **Alkalische Reiniger** | | | | | |
|---|---|---|---|---|---|
| **Zusammensetzung** | **1** | **V1** | **2** | **V2** | **V3** |
| Natriumhypochlorit | - | - | 2,0 | 2,0 | 2,0 |
| Sodium Laureth Sulfate | - | - | 4,0 | 4,0 | 4,0 |
| Sodium Laureth Phosphate | - | - | 1,0 | 1,0 | - |
| Ester gemäß Beispiel H3 | 5,0 | - | 1,0 | - | - |
| Kokosfettalkohol+2PO+10EO | - | 5,0 | - | 1,0 | 2,0 |
| Natriumhydroxid | - | - | 0,5 | 0,5 | 0,5 |
| Polyacrylat | 0,2 | 0,2 | 0,2 | 0,2 | 0,2 |

| ***Anwendungstechnische Eigenschaften*** | | | | | |
|---|---|---|---|---|---|
| Reinigungsleistung | + | - | ++ | + | + |
| Viskosität [mPas] | 200 | 90 | 240 | 180 | 150 |
| Schaumhöhe [ml] | 0 | 10 | 25 | 100 | 25 |

Man erkennt, dass die neuen Ester gegenüber den herkömmlichen Tensiden nicht nur eine höhere Reinigungsleistung besitzen, sondern auch schaumärmer sind und sich leichter verdicken lassen. Zudem besitzen sie gegenüber stark schäumenden anionischen Tensiden eine entschäumende Wirkung.

## Patentansprüche

1. Aromatische Sulfonsäurepolyglykolester der Formel **(I),** 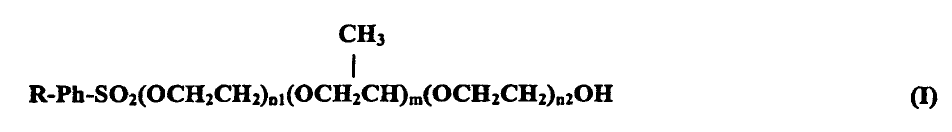 in der R für einen linearen oder verzweigten Alkylrest mit 1 bis 18 Kohlenstoffatomen, Ph für einen Phenyl- oder Naphthylrest, n1, n2 und m unabhängig voneinander für 0 der Zahlen von 1 bis 50 stehen, mit der Maßgabe, dass die Summe (n1+n2+m) von Null verschieden ist.

2. Verfahren zur Herstellung von aromatischen Sulfonsäurepolyglykolestern der Formel **(I),** 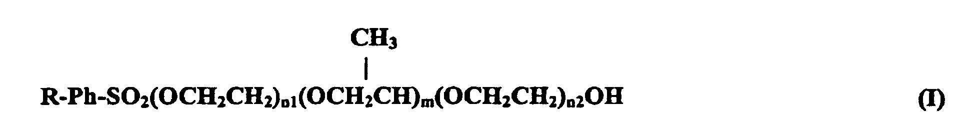 in der R für einen linearen oder verzweigten Alkylrest mit 1 bis 18 Kohlenstoffatomen, Ph für einen Phenyl- oder Naphthylrest, n1, n2 und m unabhängig voneinander für 0 der Zahlen von 1 bis 50 stehen, mit der Maßgabe, dass die Summe (n1+n2+m) von Null verschieden ist, bei dem man aromatische Sulfonsäuren der Formel **(II)**,
**R-Ph-SO**_{**3**}**H (II)**
in der R und Ph die gleichen Bedeutungen wie oben besitzen, mit Ethylenoxid und/oder Propylenoxid umsetzt.

3. Verfahren nach Anspruch 2, **dadurch gekennzeichnet, dass** man Sulfonsäuren der Formel **(II)** einsetzt, in der R für einen Alkylrest mit 10 bis 14 Kohlenstoffatomen und Ph für einen Phenylrest steht.

4. Verfahren nach Anspruch 3, **dadurch gekennzeichnet, dass** man als Sulfonsäure der Dodecylbenzolsulfonsäure einsetzt.

5. Verfahren nach mindestens einem der Ansprüche 2 bis 4, **dadurch gekennzeichnet, dass** man die aromatischen Sulfonsäuren mit durchschnittlich 1 bis 50 Mol Ethylenoxid umsetzt.

6. Verfahren nach mindestens einem der Ansprüche 2 bis 4, **dadurch gekennzeichnet, dass** man die aromatischen Sulfonsäuren mit durchschnittlich 1 bis 5 Mol Propylenoxid und anschließend mit durchschnittlich 1 bis 50 Mol Ethylenoxid umsetzt.

7. Verfahren nach mindestens einem der Ansprüche 2 bis 4, **dadurch gekennzeichnet, dass** man die aromatischen Sulfonsäuren mit einer Mischung aus durchschnittlich 1 bis 5 Mol Propylenoxid und 1 bis 50 Mol Ethylenoxid umsetzt.

8. Verwendung von aromatischen Sulfonsäuren der Formel **(I)** zur Herstellung von Spül- und Reinigungsmitteln.

9. Verwendung von aromatischen Sulfonsäuren der Formel **(I)** zur Herstellung von Textilhilfsmitteln.

10. Verwendung von aromatischen Sulfonsäuren der Formel **(I)** zur Herstellung von Lederhilfsmitteln.

## Claims

1. Aromatic sulfonic acid polyglycol esters corresponding to formula **(I):** 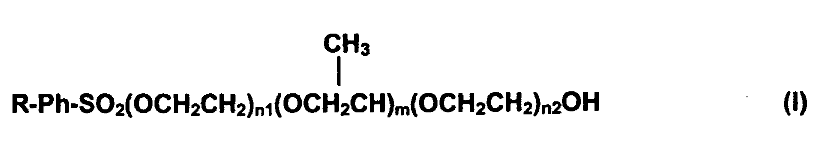 in which R is a linear or branched alkyl group containing 1 to 18 carbon atoms, Ph is a phenyl or naphthyl group, n1, n2 and m independently of one another represent 0 or numbers of 1 to 50, with the proviso that the sum (n1+n2+m) is not zero.

2. A process for the production of aromatic sulfonic acid polyglycol esters corresponding to formula **(I):** 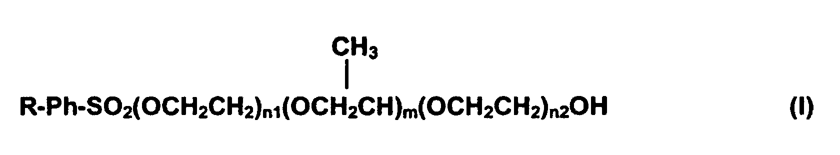 in which R is a linear or branched alkyl group containing 1 to 18 carbon atoms, Ph is a phenyl or naphthyl group, n1, n2 and m independently of one another represent 0 or numbers of 1 to 50, with the proviso that the sum (n1+n2+m) is not zero,
**characterized in that** aromatic sulfonic acids corresponding to formula **(II)**:
**R-Ph-SO**_{**3**}**H (II)**
in which R and Ph have the meanings defined above,
are reacted with ethylene oxide and/or propylene oxide.

3. A process as claimed in claim 2, **characterized in that** sulfonic acids corresponding to formula **(II)**, in which R is an alkyl group containing 10 to 14 carbon atoms and Ph is a phenyl group.

4. A process as claimed in claim 3, **characterized in that** dodecyl benzenesulfonic acid is used as the sulfonic acid.

5. A process as claimed in at least one of claims 2 to 4, **characterized in that** the aromatic sulfonic acid are reacted with, on average, 1 to 50 mol ethylene oxide.

6. A process as claimed in at least one of claims 2 to 4, **characterized in that** the aromatic sulfonic acids are reacted with, on average, 1 to 5 mol propylene oxide and then with, on average, 1 to 50 mol ethylene oxide.

7. A process as claimed in at least one of claims 2 to 4, **characterized in that** the aromatic sulfonic acids are reacted with a mixture of, on average, 1 to 5 mol propylene oxide and 1 to 50 mol ethylene oxide.

8. The use of aromatic sulfonic acids corresponding to formula **(I)** for the production of dishwashing detergents and cleaners.

9. The use of aromatic sulfonic acids corresponding to formula **(I)** for the production of textile auxiliaries.

10. The use of aromatic sulfonic acids corresponding to formula **(I)** for the production of leather auxiliaries.

## Revendications

1. Esters polyglycoliques d'acides sulfoniques aromatiques de formule (I) 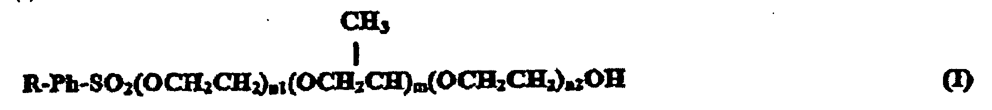 dans laquelle R représente un radical alkyle linéaire ou ramifié portant 1 à 18 atomes de carbone, Ph représente un radical phényle ou naphtyle, n1, n2 et m représentent, indépendamment les uns des autres, 0 ou des nombres de 1 à 50, étant précisé que le total (n1+n2+m) est différent de 0.

2. Procédé d'obtention d'esters polyglycoliques d'acides sulfoniques aromatiques de formule (I) 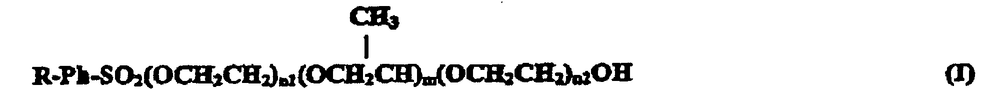 dans laquelle R représente un radical alkyle linéaire ou ramifié portant 1 à 18 atomes de carbone, Ph représente un radical phényle ou naphtyle, n1, n2 et m représentent, indépendamment les uns des autres, 0 ou des nombres de 1 à 50, étant précisé que le total (nl+n2+m) est différent de 0, selon lequel on fait réagir, avec de l'oxyde d'éthylène et/ou de l'oxyde de propylène, des acides sulfoniques aromatiques de formule (II)
**R-Ph-SO**_{**3**}**H (II)**
dans laquelle R et Ph ont les mêmes significations que ci-dessus.

3. Procédé selon la revendication 2,
**caractérisé en ce qu'**
on met en oeuvre des acides sulfoniques de formule (II) dans laquelle R représente un radical alkyle portant 10 à 14 atomes de carbone et Ph représente un radical phényle.

4. Procédé selon la revendication 3,
**caractérisé en ce qu'**
on met en oeuvre l'acide dodécylbenzène sulfonique en tant qu'acide sulfonique.

5. Procédé selon au moins l'une des revendications 2 à 4,
**caractérisé en ce qu'**
on fait réagir les acides sulfoniques aromatiques avec, en moyenne, 1 à 50 moles d'oxyde d'éthylène.

6. Procédé selon au moins l'une des revendications 2 à 4,
**caractérisé en ce qu'**
on fait réagir les acides sulfoniques aromatiques avec, en moyenne, 1 à 5 moles d'oxyde de propylène, puis avec, en moyenne, 1 à 50 moles d'oxyde d'éthylène.

7. Procédé selon au moins l'une des revendications 2 à 4,
**caractérisé en ce qu'**
on fait réagir les acides sulfoniques aromatiques avec un mélange constitué, en moyenne, de 1 à 5 moles d'oxyde de propylène et de 1 à 50 moles d'oxyde d'éthylène.

8. Utilisation d'acides sulfoniques aromatiques de formule (I), pour fabriquer des agents de rinçage et de nettoyage.

9. Utilisation d'acides sulfoniques aromatiques de formule (I), pour l'obtention d'adjuvants pour textiles.

10. Utilisation d'acides sulfoniques aromatiques de formule (I), pour l'obtention d'adjuvants pour le cuir.
